# FASCICULE DE BREVET EUROPEEN

(11) **EP 1 413 330 B1**
(45) Date de publication et mention de la délivrance du brevet: **23.01.2008**
(21) Numéro de dépôt: 03292659.4
(22) Date de dépôt: 24.10.2003
(51) Int. Cl.: A61N 1/365

(54) **Dispositif médical implantable actif de type stimulateur cardiaque, défibrillateur, cardioverteur ou dispositif multiste à gestion perfectionnée des pauses ou hypopnées respiratoires**
Aktive implantierbare medizinische Vorrichtung, wie Herzschrittmacher, Entflimmerer und/oder Mehrstellen-Einrichtung mit verbesserter Kontrolle von Atempausen oder Hypopneen
Active implantable medical device, of the type pacemaker, defibrillator, cardioverter, and/or multisite device, with improved control of respiratory pauses or hypopneas

(30) Priorité: 25.10.2002 FR 0213356
(43) Date de publication de la demande: 28.04.2004
(73) Titulaire: ELA MEDICAL, 92541 Montrouge (FR)
(72) Inventeur: Limousin, Marcel, 75014 Paris (FR); Gaggini, Guido, 20128 Milano (IT)
(74) Mandataire: Dupuis-Latour, Dominique

(56) Documents cités:
- EP-A- 0 940 155
- EP-A- 0 970 713
- EP-A- 1 151 718
- US-A- 5 974 340
- US-A- 6 141 590
- US-A1- 2003 153 953

## Description

L'invention concerne les "dispositifs médicaux actifs implantables" tels que définis par la directive 90/385/CEE du 20 juin 1990 du Conseil des communautés européennes, notamment les dispositifs tels que les stimulateurs cardiaques, défibrillateurs/cardioverteurs ou dispositifs multisite, permettant de délivrer au coeur des impulsions électriques de faible énergie pour le traitement des troubles du rythme cardiaque.

On a déjà proposé, par exemple par le EP-A-0 970 713 (ELA Médical) ou le EP-A-0 940 155 (Medtronic), de diagnostiquer et de traiter des troubles respiratoires tels que les apnées révélatrices d'une pathologie connue sous le nom de "syndrome d'apnée du sommeil" (SAS).

De façon générale, cette pathologie respiratoire est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 secondes. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure.

L'interruption ou la réduction du débit respiratoire entraînent une diminution de la concentration en oxygène du sang et des micro-réveils inconscients. Cette pathologie, qui atteint plus de 50 % des patients souffrant d'insuffisance cardiaque, a entre autres pour conséquence une somnolence diurne, une perte d'attention, un accroissement des risques d'accidents de la route, et une incidence plus élevée d'hypertension.

Le EP-A-0 970 713 ou le EP-A-0 940 155 (Medtronic) précités diagnostiquent la survenue d'une apnée à partir du signal de ventilation-minute (signal VE, dit également signal MV), qui est un paramètre à prépondérance physiologique généralement obtenu par une mesure d'impédance intrathoracique donnant une indication continue du rythme respiratoire du patient. Si une apnée survient pendant une phase de sommeil du patient (l'état de sommeil du patient pouvant être indiqué par un capteur d'activité à prépondérance physique tel qu'un accéléromètre), alors le dispositif délivre une stimulation cardiaque à une fréquence légèrement supérieure au rythme sinusal naturel du patient, ceci afin d'augmenter le débit sanguin pour pouvoir réduire l'incidence de la désaturation en oxygène consécutive au SAS.

Le point de départ de la présente invention réside dans la constatation du fait qu'une augmentation systématique par le dispositif de la fréquence cardiaque en cas de détection d'apnée ou hypopnée n'est pas toujours une mesure appropriée.

En effet, il a été rapporté que chez certains patients les apnées ou hypopnées pouvaient être suivies d'une réaction adrénergique, induisant naturellement une légère tachycardie et une augmentation significative des pressions sanguines, suffisantes pour compenser la baisse de l'activité ventilatoire.

En effet, chez ces patients, le myocarde peut réagir naturellement en adaptant sa contractilité de manière à accroître le débit sanguin et, ainsi, maintenir le sang sensiblement au même niveau de saturation en oxygène.

Idéalement, pour décider s'il y a lieu ou non d'appliquer au myocarde une stimulation à une fréquence supérieure au rythme sinusal naturel du patient, le meilleur critère serait une mesure de la saturation en oxygène dans le sang, la stimulation n'étant déclenchée qu'en cas de désaturation avérée et significative.

Mais cette mesure directe est difficile à réaliser de manière simple et permanente dans le contexte d'un dispositif implanté.

Pour pallier cette difficulté, et traiter avec discernement les apnées et hypopnées, l'invention propose, essentiellement, d'estimer les variations de contractilité du myocarde au moyen d'un capteur hémodynamique.

En cas d'anomalie respiratoire détectée (apnée ou hypopnée), avant toute action le dispositif estime, préalablement ou postérieurement, s'il y a eu, ou non, modification de contractilité corrélative :
- si le capteur indique la survenue d'une chute hémodynamique notable, révélant ainsi que le myocarde n'a pas pu adapter naturellement sa contractilité suite à l'anomalie, ou ne l'a pas assez adaptée, alors le dispositif prend une action (par exemple, si la chute de contractilité fait suite à une apnée ou à une hypopnée, stimulation à une fréquence supérieure au rythme sinusal naturel), afin de compenser la désaturation en oxygène induite par le trouble respiratoire ;
- dans le cas contraire, c'est-à-dire si le capteur délivre une information hémodynamique stable ou peu évolutive, le dispositif ne prend pas d'action, car cette situation révèle vraisemblablement qu'il n'y a pas eu de désaturation nécessitant une récupération par augmentation du débit.

En d'autres termes, l'invention propose, en cas d'anomalie respiratoire détectée, d'adapter la réaction du dispositif en fonction de la stabilisation de l'information hémodynamique, qui fournit une estimation des variations de contractilité, corrélées aux augmentations de pression sanguine.

Plus précisément, le dispositif de l'invention est du type décrit par les EP-A-0 970 713 ou EP-A-0 940 155 précités, c'est-à-dire comprenant des moyens de mesure de l'activité respiratoire, aptes à délivrer un signal d'activité ventilatoire du patient et des moyens d'analyse du signal d'activité ventilatoire, aptes à détecter la survenue d'apnées ou d'hypopnées respiratoires.

De façon caractéristique de l'invention, le dispositif comprend : des moyens de mesure d'accélération endocardiaque ou d'impédance intracardiaque, aptes à délivrer un signal hémodynamique représentatif de la contractilité du myocarde ; des moyens d'analyse du signal hémodynamique, aptes à détecter la survenue d'une variation de ce signal hémodynamique et à déterminer le caractère significatif, ou non, de cette variation ; et des moyens de modification conditionnelle d'un paramètre de fonctionnement du dispositif en cas de variation significative du signal hémodynamique détectée en relation avec la détection d'une apnée ou d'une hypopnée.

Les moyens d'analyse du signal hémodynamique peuvent déterminer le caractère significatif ou non d'une variation du signal hémodynamique soit après, soit avant détection d'une apnée ou d'une hypopnée. En effet, le dispositif ne peut, par définition, détecter la présence d'une apnée qu'à l'issue d'une période relativement longue (typiquement 10 secondes), mais une variation de la contractilité peut avoir été détectée au cours de cette période où l'apnée, simplement suspectée, n'est pas encore véritablement confirmée.

D'autres caractéristiques subsidiaires avantageuses sont visées dans les sous-revendications.

On va maintenant décrire un exemple de mise en oeuvre de l'invention, en référence aux dessins annexés.

La figure 1 est un organigramme général d'un premier mode de mise en oeuvre de l'invention.

La figure 2 est homologue de la figure 1, pour une autre forme de mise en oeuvre.

Le dispositif de l'invention comporte des moyens pour détecter la survenue d'apnées ou d'hypopnées par analyse du rythme respiratoire du patient pendant son sommeil, ce rythme étant donné par l'évolution au cours du temps du signal de ventilation-minute (signal MV).

L'invention peut notamment être appliquée aux dispositifs commercialisé par ELA Médical, Montrouge, France. Il s'agit de dispositifs à microprocesseur comportant des circuits pour recevoir, mettre en forme et traiter des signaux électriques recueillis par des électrodes implantées. Ils est possible d'y transmettre par télémétrie des logiciels qui seront conservés en mémoire et exécutés pour mettre en oeuvre les fonctions de l'invention qui seront décrites ci-dessous.

Le signal MV est un paramètre à prépondérance physiologique obtenu par une mesure intrathoracique d'impédance. Cette mesure est opérée entre deux électrodes disposées dans la cage thoracique, ou entre une électrode (par exemple une électrode de stimulation, si le dispositif implanté est un stimulateur cardiaque) et le boîtier du dispositif. L'impédance est mesurée par injection d'un courant constant de quelques centaines de micro-ampères, à une fréquence de quelques hertz, typiquement 8 Hz. Cette technique est par exemple décrite par Bonnet JL et coll., Measurement of Minute-Ventilation with Different DDDR Pacemaker Electrode Configurations, *PACE,* Vol. 21, 98, Part 1, et elle est mise en oeuvre dans les appareils de la série *Chorus RM 7034* d'ELA Médical.

Par ailleurs, le dispositif comporte des moyens pour détecter les phases de sommeil du patient, afin de ne procéder à le recherche des apnées ou hypopnées que pendant les phases de sommeil, car les variations d'activité respiratoire survenant pendant une phase d'éveil ne sont normalement pas pathologiques.

La période de sommeil est diagnostiquée par exemple par un capteur physiologique de mesure de la ventilation-minute, éventuellement combiné à un capteur d'activité mesurant un paramètre à prépondérance physique tel que l'accélération. Les EP-A-0 750 920, EP-A-0 770 407 et EP-A-1 317 943, tous trois au nom d'ELA Médical, décrivent diverses techniques de discrimination des périodes de sommeil et d'éveil qui sont applicables dans le cadre de la présente invention.

Le dispositif considère qu'il y a apnée lorsqu'il détecte un arrêt respiratoire de durée supérieure à 10 secondes, phénomène qu'il est aisé de détecter par suivi du signal MV. Pour détecter les hypopnées, le dispositif peut par exemple comparer entre elles des moyennes glissantes du signal MV, moyennes établies par exemple sur une durée de 10 secondes : si, entre deux moyennes consécutives, une décroissance importante de la ventilation-minute est détectée, par exemple de plus de 50 %, alors le dispositif considère qu'il y a eu hypopnée.

Au surplus, pour permettre la mise en oeuvre de l'invention, le dispositif comporte un capteur hémodynamique permettant d'estimer les variations de contractilité, qui sont corrélées aux augmentations de pression sanguine.

Ce paramètre hémodynamique est plus sensible et plus rapide que la mesure des variations de fréquence cardiaque, pour estimer les conséquences de la désaturation en oxygène.

Le capteur hémodynamique peut être un capteur d'accélération endocardique de type PEA (*Peak Endocardial Acceleration*) comme décrit par exemple dans les EP-A 0 515 319, EP-A 0 582 162 ou EP-A 0 655 260 (tous trois au nom de Sorin Biomedica Cardio SpA).

Le capteur hémodynamique peut être également un capteur d'impédance endocavitaire, par exemple un capteur de mesure de bioimpédance trans-valvulaire, comme décrit par le EP-A 1 116 497, ou de bioimpédance transseptale oblique, comme décrit par le EP-A 1 138 346 (tous deux au nom d'ELA Médical).

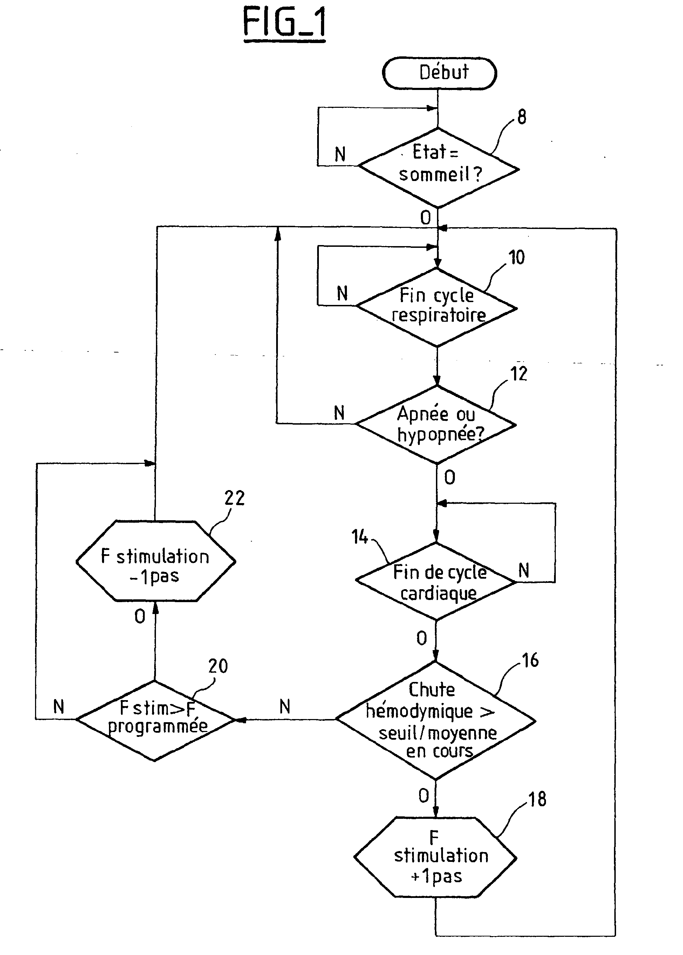

On va maintenant décrire plus précisément, en référence à l'organigramme de la figure 1, la manière dont l'invention peut être mise en oeuvre.

Le dispositif, après avoir attendu la fin d'un cycle respiratoire (étape 10), détermine (étape 12) si une apnée ou une hypopnée est survenue, c'est-à-dire s'il a trouvé un cycle respiratoire de durée supérieure à 10 secondes ou bien une chute du débit respiratoire supérieure à 50 %.

Dans la négative, aucune action n'est prise et le dispositif continue à surveiller le rythme respiratoire.

Dans l'affirmative, en revanche, le dispositif analyse s'il y a eu, ou non, une variation significative de l'état hémodynamique consécutivement à cette apnée ou hypopnée.

A cet effet, après avoir attendu la fin du cycle cardiaque courant (étape 14), le dispositif détermine (étape 16) si le capteur hémodynamique a détecté une chute du signal hémodynamique supérieure à un seuil de référence donné. Ce seuil de référence est de préférence un seuil dynamique constitué par exemple par la moyenne, sur une pluralité de cycles cardiaques, des valeurs de signal délivrées par le capteur hémodynamique ; la moyenne considérée est bien entendu basée sur des valeurs de signal antérieures au cycle respiratoire présentant l'anomalie, c'est-à-dire le cycle respiratoire dont on a attendu la fin à l'étape 10.

En cas de chute hémodynamique avérée, qui laisse supposer une diminution du taux d'oxygène dans le sang du fait de l'apnée ou de l'hypopnée, alors le dispositif augmente d'un pas (étape 18) la fréquence de stimulation. Cette augmentation légère de la fréquence (le pas d'augmentation étant typiquement de 1 à 5 cpm) permet de compenser la désaturation en oxygène.

En variante ou en complément de la fréquence cardiaque, d'autres paramètres de fonctionnement du stimulateur peuvent être modifiés : on peut ainsi envisager par exemple un raccourcissement du délai AV et/ou la mise en route d'une stimulation multisite (le EP-A-0 0925 806 (ELA Medical) décrit un tel dispositif multisite configurable auquel peuvent être appliqués les enseignements de l'invention).

L'algorithme est ensuite répété de la même façon que précédemment.

L'augmentation de la fréquence de simulation à l'étape 18 a normalement eu pour conséquence d'améliorer l'état hémodynamique du patient. Si cette amélioration n'est pas suffisante pour aboutir à une stabilisation de l'information hémodynamique, le dispositif détectera encore une chute hémodynamique (à l'étape 16 de l'itération suivante) et augmentera d'un pas supplémentaire la fréquence de stimulation (à l'étape 18 subséquente).

En revanche, si l'augmentation de la fréquence de stimulation, d'un ou plusieurs pas (c'est-à-dire après une ou plusieurs itérations), a conduit à une stabilisation de l'état hémodynamique, une absence de variation significative du signal, détectée à l'étape 16, conduira au test de l'étape 20 où le dispositif compare la fréquence de stimulation courante à une fréquence de référence, préprogrammée.

Si la fréquence de stimulation est supérieure à cette fréquence préprogrammée, alors (étape 22) le dispositif diminue d'un pas la fréquence de stimulation avant de retourner au point de départ de l'organigramme.

Ainsi, par ajustements successifs de pas, en plus ou en moins, la fréquence de stimulation pourra être réglée en permanence au minimum efficace permettant d'aboutir à une stabilisation précise de l'état hémodynamique, sans dépasser cette valeur de plus d'un pas.

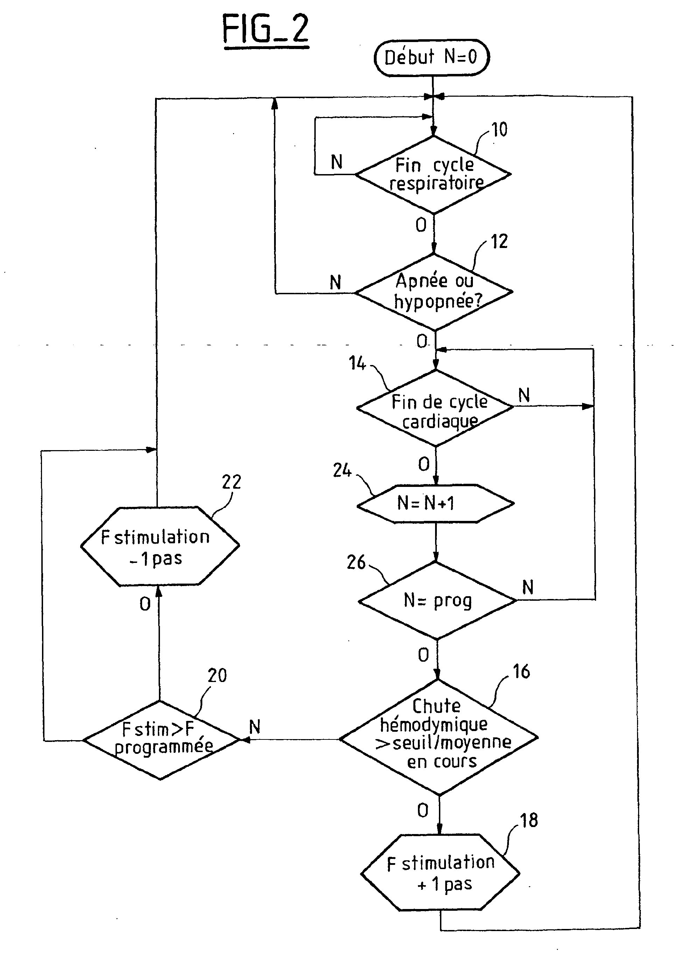

La figure 2 illustre une variante de l'organigramme de la figure 2.

Dans cette variante, le dispositif opère le test de l'étape 16 sur le signal hémodynamique non pas sur le cycle cardiaque qui suit immédiatement l'apnée ou l'hypopnée détectée à l'étape 12, mais seulement après N cycles cardiaques ayant suivi cette apnée ou cette hypopnée.

À cet effet, l'algorithme compte le nombre de cycles écoulés (étape 24, compteur N) et compare la valeur de ce compteur à une valeur programmée (étape 26), par exemple N = 5 cycles.

Ceci permet de mieux laisser s'exprimer la réaction adrénergique éventuelle susceptible d'induire une modification significative de l'état hémodynamique.

## Revendications

1. Un dispositif médical implantable actif du type stimulateur cardiaque, défibrillateur, cardioverteur ou dispositif multisite, comprenant :
- des moyens de mesure de l'activité respiratoire, aptes à délivrer un signal d'activité ventilatoire du patient, et
- des moyens d'analyse du signal d'activité ventilatoire, aptes à détecter la survenue d'apnées ou d'hypopnées respiratoires,
**caractérisé en ce qu**'il comprend:
- des moyens de mesure d'accélération endocardiaque ou d'impédance intracardiaque, aptes à délivrer un signal hémodynamique représentatif de la contractilité du myocarde,
- des moyens d'analyse du signal hémodynamique, aptes à détecter la survenue d'une variation de ce signal hémodynamique et à déterminer le caractère significatif, ou non, de cette variation, et
- des moyens de modification conditionnelle d'un paramètre de fonctionnement du dispositif en cas de variation significative du signal hémodynamique détectée en relation avec la détection d'une apnée ou d'une hypopnée.

2. Le dispositif de la revendication 1, dans lequel les moyens d'analyse du signal hémodynamique déterminent le caractère significatif ou non d'une variation du signal hémodynamique après détection d'une apnée ou d'une hypopnée.

3. Le dispositif de la revendication 1, dans lequel les moyens d'analyse du signal hémodynamique déterminent le caractère significatif ou non d'une variation du signal hémodynamique avant détection d'une apnée ou d'une hypopnée.

4. Le dispositif de la revendication 1, dans lequel les moyens de modification modifient de manière temporaire ledit paramètre de fonctionnement du dispositif.

5. Le dispositif de la revendication 4, dans lequel les moyens de modification rétablissent ledit paramètre de fonctionnement du dispositif à sa valeur antérieure lorsque les moyens d'analyse du signal hémodynamique ne détectent plus de variation significative du signal hémodynamique.

6. Le dispositif de la revendication 1, dans lequel ledit paramètre de fonctionnement du dispositif modifié conditionnellement est la fréquence de stimulation, cette fréquence étant augmentée en cas de variation significative du signal hémodynamique détectée en relation avec la détection d'une apnée ou d'une hypopnée.

7. Le dispositif de la revendication 1, dans lequel ledit paramètre de fonctionnement du dispositif modifié conditionnellement est le délai atrio-ventriculaire, ce délai étant raccourci en cas de variation significative du signal hémodynamique détectée en relation avec la détection d'une apnée ou d'une hypopnée.

8. Le dispositif de la revendication 1, dans lequel ledit paramètre de fonctionnement du dispositif modifié conditionnellement est le mode de stimulation, les moyens de modification déclenchant une stimulation multisite en cas de variation significative du signal hémodynamique détectée en relation avec la détection d'une apnée ou d'une hypopnée.

9. Le dispositif de la revendication 2, dans lequel les moyens d'analyse du signal hémodynamique sont des moyens aptes à comparer le signal hémodynamique mesuré lors du cycle cardiaque suivant le cycle respiratoire au cours duquel est survenue l'apnée ou l'hypopnée, avec une moyenne des signaux hémodynamiques antérieurs à ce cycle respiratoire au cours duquel est survenue l'apnée ou l'hypopnée.

10. Le dispositif de la revendication 2, dans lequel les moyens d'analyse du signal hémodynamique sont des moyens aptes à comparer le signal hémodynamique mesuré après une pluralité de cycles cardiaques suivant le cycle respiratoire au cours duquel est survenue l'apnée ou l'hypopnée, avec une moyenne des signaux hémodynamiques antérieurs à ce cycle respiratoire au cours duquel est survenue l'apnée ou l'hypopnée.

## Claims

1. An active implantable medical device of the cardiac pacemaker, defibrillator, cardioverter or multisite device type, comprising:
- means for measuring respiratory activity, able to deliver a patient's ventilatory activity signal, and
- means for analyzing the ventilatory activity signal, able to detect the occurrence of respiratory apneas or hypopneas,
**characterised in that** it comprises:
- means for measuring endocardial acceleration or intracardiac impedance, able to deliver a hemodynamic signal representative of the contractility of the myocardium,
- means for analyzing the hemodynamic signal, able to detect the occurrence of a variation of this hemodynamic signal and to determine the significant nature, yes or no, of this variation, and
- means for the conditional modification of an operating parameter of the device in case of a significant variation of the hemodynamic signal detected in relation to the detection of apnea or hypopnea.

2. The device of claim 1, wherein the means for analyzing the hemodynamic signal determine the significant nature, yes or no, of a variation of the hemodynamic signal after the detection of apnea or hypopnea.

3. The device of claim 1, wherein the means for analyzing the hemodynamic signal determine the significant nature, yes or no, of a variation of the hemodynamic signal before the detection of apnea or hypopnea.

4. The device of claim 1, wherein the modification means modify said operating parameter of the device in a temporary manner.

5. The device of claim 4, wherein the modification means restore said operating parameter of the device to its previous value when the means for analysing the hemodynamic signal no longer detect a significant variation of the hemodynamic signal.

6. The device of claim 1, wherein said conditionally modified operating parameter of the device is the stimulation frequency, this frequency being increased in case of a significant variation of the hemodynamic signal detected in relation to the detection of apnea or hypopnea.

7. The device of claim 1, wherein said conditionally modified operating parameter of the device is the atrioventricular delay, this delay being shortened in case of a significant variation of the hemodynamic signal detected in relation to the detection of apnea or hypopnea.

8. The device of claim 1, wherein said conditionally modified operating parameter of the device is the stimulation mode, the modification means triggering a multisite stimulation in case of a significant variation of the hemodynamic signal detected in relation to the detection of apnea or hypopnea.

9. The device of claim 2, wherein the hemodynamic signal analyzing means are means able to compare the hemodynamic signal measured during the cardiac cycle following the respiratory cycle during which apnea or hypopnea occurred with an average of the hemodynamic signals prior to this respiratory cycle during which apnea or hypopnea occurred.

10. The device of claim 2, wherein the hemodynamic signal analyzing means are means able to compare the hemodynamic signal measured after a plurality of cardiac cycles following the respiratory cycle during which apnea or hypopnea occurred with an average of the hemodynamic signals prior to this respiratory cycle during which apnea or hypopnea occurred.

## Patentansprüche

1. Aktive implantierbare medizinische Vorrichtung vom Typ eines Herzschrittmachers, Defibrillators, Kardioverters oder einer Multisite-Vorrichtung mit:
- Mitteln zur Messung der Atmungsaktivität, die geeignet sind, ein Signal der Atmungsaktivität des Patienten zu liefern, und
- Mitteln zur Analyse des Signals der Atmungsaktivität, die geeignet sind, das Auftreten von respiratorischen Apnoen oder Hypopnoen zu erfassen,
**dadurch gekennzeichnet, dass** sie folgendes umfasst:
- Mittel zur Messung einer endokardialen Beschleunigung oder einer intrakardialen Impedanz, die geeignet sind, ein hämodynamisches Signal zu liefern, das für die Kontraktilität des Herzmuskels repräsentativ ist,
- Mittel zur Analyse des hämodynamischen Signals, die geeignet sind, das Auftreten einer Veränderung dieses hämodynamischen Signals zu erfassen und den signifikativen Charakter, ja oder nein, dieser Veränderung festzustellen, und
- Mittel zur bedingungsabhängigen Modifizierung eines Betriebsparameters der Vorrichtung im Falle einer signifikativen Veränderung des hämodynamischen Signals, die in Bezug zu der Erfassung einer Apnoe oder einer Hypopnoe erfasst wird.

2. Vorrichtung nach Anspruch 1, bei welcher die Mittel zur Analyse des hämodynamischen Signals den signifikativen Charakter, ja oder nein, einer Veränderung des hämodynamischen Signals nach Erfassung einer Apnoe oder einer Hypopnoe feststellen.

3. Vorrichtung nach Anspruch 1, bei welcher die Mittel zur Analyse des hämodynamischen Signals den signifikativen Charakter, ja oder nein, einer Veränderung des hämodynamischen Signals vor Erfassung einer Apnoe oder einer Hypopnoe feststellen.

4. Vorrichtung nach Anspruch 1, bei welcher die Modifizierungsmittel den Betriebsparameter der Vorrichtung vorübergehend modifizieren.

5. Vorrichtung nach Anspruch 4, bei welcher die Modifizierungsmittel den Betriebsparameter der Vorrichtung auf seinen vorherigen Wert zurücksetzen, wenn die Mittel zur Analyse des hämodynamischen Signals keine signifikative Veränderung des hämodynamischen Signals mehr erfassen.

6. Vorrichtung nach Anspruch 1, bei welcher der bedingungsabhängig modifizierte Betriebsparameter der Vorrichtung die Stimulationsfrequenz ist, wobei diese Frequenz im Falle einer signifikativen Veränderung des hämodynamischen Signals, die in Bezug zu der Erfassung einer Apnoe oder einer Hypopnoe erfasst wird, erhöht wird.

7. Vorrichtung nach Anspruch 1, bei welcher der bedingungsabhängig modifizierte Betriebsparameter der Vorrichtung die atrioventrikuläre Verzögerung ist, wobei diese Verzögerung im Falle einer signifikativen Veränderung des hämodynamischen Signals, die in Bezug zu der Erfassung einer Apnoe oder einer Hypopnoe erfasst wird, verkürzt wird.

8. Vorrichtung nach Anspruch 1, bei welcher der bedingungsabhängig modifizierte Betriebsparameter der Vorrichtung der Stimulationsmodus ist, wobei die Modifizierungsmittel im Falle einer signifikativen Veränderung des hämodynamischen Signals, die in Bezug zu der Erfassung einer Apnoe oder einer Hypopnoe erfasst wird, eine Multisite-Stimulation auslösen.

9. Vorrichtung nach Anspruch 2, bei welcher die Mittel zur Analyse des hämodynamischen Signals Mittel sind, die geeignet sind, das hämodynamische Signal, das während des Herzzyklus gemessen wurde, welcher dem Atmungszyklus folgt, während welchem die Apnoe oder Hypopnoe aufgetreten ist, mit einem Durchschnitt der hämodynamischen Signale zu vergleichen, die diesem Atmungszyklus vorangingen, während welchem die Apnoe oder Hypopnoe aufgetreten ist.

10. Vorrichtung nach Anspruch 2, bei welcher die Mittel zur Analyse des hämodynamischen Signals Mittel sind, die geeignet sind, das hämodynamische Signal, das nach einer Mehrzahl von Herzzyklen gemessen wurde, welche dem Atmungszyklus folgen, während welchem die Apnoe oder Hypopnoe aufgetreten ist, mit einem Durchschnitt der hämodynamischen Signale zu vergleichen, die diesem Atmungszyklus vorangingen, während welchem die Apnoe oder Hypopnoe aufgetreten ist.
